# EUROPEAN PATENT SPECIFICATION

(11) **EP 2 109 442 B1**
(45) Date of publication and mention of the grant of the patent: **26.03.2014**
(21) Application number: 08729350.2
(22) Date of filing: 08.02.2008
(51) Int. Cl.: A61K 9/08, A61K 47/32, A61K 47/36, A61P 27/04

(54) **OPHTHALMIC COMPOSITIONS CONTAINING A SYNERGISTIC COMBINATION OF THREE POLYMERS**
AUGENMEDIZINISCHE ZUSAMMENSETZUNGEN MIT SYNERGISTISCHER KOMBINATION AUS DREI POLYMEREN
COMPOSITIONS OPHTALMIQUES COMPRENANT UNE COMBINAISON SYNERGIQUE DE TROIS POLYMÈRES

(30) Priority: 09.02.2007 US 888975 P
(43) Date of publication of application: 21.10.2009
(73) Proprietor: Novartis AG, 4056 Basel (CH)
(72) Inventor: CHOWHAN, Masood, A., Arlington, Texas 76016 (US); CHEN, Huagang, Arlington, TX 76016 (US)
(74) Representative: Teipel, Stephan
(86) International application number: PCT/US2008/053378
(87) International publication number: WO 2008/100807

(56) References cited:
- EP-A- 0 663 208
- WO-A-2004/112836
- US-A1- 2004 253 202
- A. ZIMMER ET AL.: "Evaluation of pilocarpine loaded albumin particles as controlled drug delivery systems for the eye. II Co-administration with bioadhesive and viscous polymers" JOURNAL OF CONTROLLED RELEASE, vol. 33, 1995, pages 31-46, XP004037639
- DATABASE CA CHEMICAL ABSTRACTS SERVICE, COLUMBUS, OHIO, US; XP002501200 retrieved from STN Database accession no. 140:229298 & T, MURAKAMI ET AL,: "Combined effects of hyaluronan and artificial tear solution in rat dry eye model." ATARASHII GANKA, vol. 21, no. 1, 2004, pages 87-90, JP
- DATABASE BIOSIS [Online] BIOSCIENCES INFORMATION SERVICE, PHILADELPHIA, PA, US; XP002501201 retrieved from EPOPROGS Database accession no. PREV200510251678 & M. GUILLON ET AL.: "Effect of eye drop compositional characteristics on tear film." ANNUAL MEETING OF THE ASSOCIATION FOR RESEARCH IN VISION AND OPHTHALMOLOGY, FORT LAUDERDALE, FL, 2004, vol. 45, 2004, page U327,
- S. BERNATCHEZ ET AL.: "Sodium hyaluronate 0.25% used as a vehicle increases the bioavailability of topically administered gentamicin." GRAEFE'S ARCHIVE FOR CLINICAL AND EXPERIMENTAL OPHTHALMOLOGY, vol. 231, no. 3, 1993, pages 157-161, XP008097227
- MALTESE ET AL: "Novel polysaccharides-based viscoelastic formulations for ophthalmic surgery: Rheological characterization", BIOMATERIALS, ELSEVIER SCIENCE PUBLISHERS BV., BARKING, GB, vol. 27, no. 29, 1 October 2006 (2006-10-01), pages 5134-5142, XP005518115, ISSN: 0142-9612, DOI: 10.1016/J.BIOMATERIALS.2006.05.036

## Description

### BACKGROUND OF THE INVENTION

### 1. Field of the Invention

The present invention relates to pharmaceutical compositions. In particular, this invention relates to topically administrable ophthalmic compositions that contain three polymeric components.

### 2. Description of Related Art

The use of polymeric ingredients in topically administrable ophthalmic compositions is well known. Polymeric ingredients are typically used in suspension compositions as physical stability aids, helping to keep the insoluble ingredients suspended or easily redispersible. In solution compositions, polymeric ingredients are typically used to increase the composition's viscosity.

Many polymers have been used in topically administrable ophthalmic compositions. Included among these are cellulosic polymers, such as hydroxypropyl methylcellulose, hydroxyethyl cellulose, and ethylhydroxyethyl cellulose. Also included are synthetic polymers, such as carboxyvinyl polymers and polyvinyl alcohol. Still others include polysaccharides such as xanthan gum, guar gum, and dextran.

Combinations of polymers have also been used in ophthalmic compositions. Certain combinations of polymers are known to provide synergistic effects on viscosity and, in some cases, even a phase transition from a liquid to a gel. For example, U.S. Patent No. 4,136,173 discloses ophthalmic compositions containing a combination of xanthan gum and locust bean gum.

WO 2004/112836 discloses ophthalmic compositions comprising synergistic combinations of two polymers, US 2004/0253202 synergistic combinations of three polymers.

One approach to achieving a target viscosity in a topically administrable ophthalmic composition might involve simply adding a sufficient amount of one polymeric ingredient. Often, however, it is desirable to minimize the total amount of polymeric additives in topically administrable ophthalmic compositions. A mixed polymer system containing more than one polymer can significantly enhance the viscosity and lubrication property of a composition while minimizing total polymer concentration and cost of materials.

### SUMMARY OF THE INVENTION

The present invention is directed toward ophthalmic compositions that contain three polymeric components. More specifically, the present invention is directed toward an aqueous composition suitable for ophthalmic administration comprising three polymeric ingredients having a synergistic effect on the composition's viscosity which is greater than 150% of the simple sum of the viscosities of the three respective simple polymer solutions and which is determined using a Brookfield cone/plate viscosimeter with number 42 cone/plate set (30 rpm, at 25°C) for samples with viscosity less than 20 cps and (6 rpm, at 25°C) for samples with viscosities between 20-50 cps and number 52 cone/plate set (6 rpm, at 25°C) for samples with viscosity more than 50 cps, wherein the three polymeric ingredients are a) hyaluronic acid, b) guar gum, and c) hydroxypropyl methylcellulose or a carboxyvinyl polymer, provided that if the composition comprises a carboxyvinyl polymer then the composition does not contain sodium chloride or boric acid, wherein the three polymeric ingredients are present in a ratio of 1:1:1 to 3:3:3, and the total concentration of the three polymeric ingredients ranges from 0.1 - 1% (w/v). The compositions are useful as artificial tear products, but can also serve as vehicles for delivering ophthalmic drugs.

The present invention is based upon the finding that the specified combinations of three polymers have a synergistic effect on viscosity.

### BRIEF DESCRIPTION OF THE DRAWINGS

Fig. 1 shows the synergistic effect on viscosity of a combination of sodium hyaluronate, guar gum and carbopol.
Fig. 2 shows the synergistic effect on viscosity of a combination of sodium hyaluronate, guar gum and hydroxypropyl methylcellulose.
Fig. 3 shows the effect of total polymer concentration on viscosity for combinations of sodium hyaluronate and guar gum with carbopol or hydroxypropyl methylcellulose.

### DETAILED DESCRIPTION OF THE INVENTION

Unless otherwise indicated, all ingredient concentrations are listed as a weight/ volume percentage basis (%w/v).

The ophthalmic compositions of the present invention are aqueous compositions that include a combination of three polymeric ingredients: hyaluronic acid ("HA"), guar gum ("Guar"), and either hydroxypropyl methylcellulose ("HPMC") or a carboxyvinyl polymer ("carbomer"). All of these types of polymers are known and have been used in ophthalmic compositions. All of these types of polymers are also commercially available.

Hyaluronic acid is commercially available from a variety of sources, including Genzyme and Hyaluron Inc. Hyaluronic acid is available in many grades, with molecular weights ranging from 100,000 to greater than 3 million dalton. As used herein, hyaluronic acid also encompasses the sodium salt form of hyaluronic acid, known as sodium hyaluronate, which is also commercially available.

Guar includes guar gum and guar gum derivatives, such as the hydroxypropyl or hydroxypropyltrimonium chloride derivatives of guar gum. Guar and its derivatives are described in U.S. Patent No. 6,316,506, the entire contents of which are hereby incorporated by reference. For purposes of the present application, guar includes unsubstituted guar gum and its substituted derivatives. Guar gum and many of its derivatives are commercially available from Rhone-Poulenc (Cranbury, New Jersey), Hercules, Inc. (Wilmington, Delaware) and TIC Gum, Inc. (Belcamp, Maryland). A preferred derivative for use in the compositions of the present invention is hydroxypropyl guar ("HP-Guar"). The concentration of guar in the compositions of the present invention will generally range from 0.01 - 0.2 %, and will preferably be 0.1 %.

HPMC is commercially available from the Dow Chemical Company under the brand name Methocel^{®}. HPMC is available in a variety of grades. Most preferred for use in the compositions of the present invention is Methocel E4M, (HPMC 2910), which has a number average molecular weight of approximately 86,000 dalton. The concentration of HPMC in the compositions of the present invention will generally range from 0.05 - 0.5 %, and will preferably be 0.3 %.

Carboxyvinyl polymers suitable for use in the present invention are also known as "carbomers" or carboxypolymethylene. They are commercially available from sources such as Noveon, Inc. (Cleveland, Ohio), which distributes them under the trade name Carbopol^{®}. Carbopol polymers are crosslinked, acrylic acid-based polymers. They are cross-linked with allyl sucrose or allylpentaerythritol. Carbopol copolymers are polymers of acrylic acid, modified by C₁₀₋₃₀ alkyl acrylates, and crosslinked with allylpentaerythritol. A preferred carbomer for use in the compositions of the present invention is a polymer of acrylic acid cross-linked with allyl sucrose or allylpentaerythritol, which is commercially available as Carbopol^{®} 974P. The concentration of carbomer in the compositions of the present invention will generally range from 0.01 - 0.2 %, and will preferably be 0.1 %.

The aqueous compositions of the present invention contain the three specified polymeric ingredients in a ratio ranging from 1:1:1 to 3:3:3, with a ratio of 3:1:1 being most preferred, where the amount of HA is listed first, the amount of Guar is listed second, and the amount of either HPMC or carbomer is listed third. The total concentration of the three polymeric ingredients should range from 0.1 - 1%, preferably 0.3 - 0.9%, and most preferably, 0.3 - 0.7%.

In addition to the three required polymeric ingredients, the aqueous compositions of the present invention may contain other ingredients as excipients. For example, the compositions may include one or more pharmaceutically acceptable buffering agents, preservatives (including preservative adjuncts), tonicity-adjusting agents, surfactants, solubilizing agents, stabilizing agents, comfort-enhancing agents, emollients, pH-adjusting agents and/or lubricants. Preferably, the aqueous composition does not contain any polymeric ingredients, other than the synergistic combination of the three polymeric ingredients specified above, with the exception of polymeric preservatives for compositions that contain a preservative. If the compositions contain a carbomer, then the compositions of the present invention do not contain any ionic tonicity-adjusting agents, such as sodium chloride, or other ionic excipients, such as boric acid, as these ingredients have a significant, detrimental effect on the composition's viscosity.

The compositions of the invention preferably have a pH in the range of 4 - 9, preferably 6 - 8, and most preferably 6.5- 7.5 If the compositions contain a carbomer as one of the three polymers, it is critical that the compositions are formulated so that the target pH is not exceeded. Once a target pH has been exceeded in compositions containing a carbomer, adding an acid such as hydrochloric acid to adjust the pH downward can compromise the synergistic viscosity. It is known that relatively small amounts of acid or salts, on the order of 0.005%, can have a significant effect on the viscosity of compositions containing a carbomer.

The compositions of the present invention preferably have an osmolality in the range of 220 - 340 mOsm/kg, and preferably have an osmolality in the range of 235 - 300 mOsm/kg.

The aqueous compositions of the present invention are suitable for use as artificial tear products to relieve symptoms of dry eye. Alternatively, the compositions of the present invention may act as a vehicle for an ophthalmic drug. Ophthalmic drugs suitable for use in the compositions of the present invention include, but are not limited to: anti-glaucoma agents, such as beta-blockers including timolol, betaxolol, levobetaxolol, carteolol, miotics including pilocarpine, carbonic anhydrase inhibitors, prostaglandins, seretonergics, muscarinics, dopaminergic agonists, adrenergic agonists including apraclonidine and brimonidine; anti-angiogenesis agents; anti-infective agents including quinolones such as ciprofloxacin, and aminoglycosides such as tobramycin and gentamicin; non-steroidal and steroidal anti-inflammatory agents, such as suprofen, diclofenac, ketorolac, rimexolone and tetrahydrocortisol; growth factors, such as EGF; immunosuppressant agents; and anti-allergic agents including olopatadine. The ophthalmic drug may be present in the form of a pharmaceutically acceptable salt, such as timolol maleate, brimonidine tartrate or sodium diclofenac. Compositions of the present invention may also include combinations of ophthalmic drugs, such as combinations of (i) a beta-blocker selected from the group consisting of betaxolol and timolol, and (ii) a prostaglandin selected from the group consisting of latanoprost; 15-keto latanoprost; travoprost; and unoprostone isopropyl. In the case of a cationic drug, the amount of drug and/or the amount of carboxyvinyl polymer and/or the identity and amount of other formulation ingredients may need to be adjusted to minimize or eliminate interactions between the carboxyvinyl polymer and the cationic drug. Preferably, the ophthalmic drug is a neutral or negatively-charged drug.

Although the amount of drug included in the compositions of the present invention will be whatever amount is therapeutically effective and will depend upon a number of factors, including the identity and potency of the chosen drug, the total concentration of drug will generally be about 5% or less.

The compositions of the present invention are preferably not formulated as solutions that undergo a phase transition to a gel upon administration to the eye. The compositions illustrated in the Examples below do not gel upon administration to the eye.

The compositions of the present invention may be topically applied to the eye or injected into the eye, depending upon the target site and disease or condition to be treated. To treat diseases or conditions inside the eye rather than at the surface of the eye, the compositions of the present invention may, for example, be administered by intravitreal injection, subconjunctival injection, sub-tenon injection, retrobulbar injection, suprachoroidal injection, or periocular injection. A syringe apparatus including an appropriately sized needle, for example, a 27 gauge needle or a 30 gauge needle, can be effectively used to inject the composition into the posterior segment of an eye of a human or animal. The combination of polymers may be particularly advantageous for injections into the eye for the following reasons: prevention of reflux, prolonged duration of action so as to increase the period of time between repeat injections, and reduction in the total amount of polymer required to achieve a target viscosity, thereby reducing the polymer disposition from back of the eye.

The following examples are presented to illustrate further various aspects of the present invention.

### EXAMPLES

### Example 1: Artificial Tear Composition

A representative formulation for an artificial tear product according to the present invention is shown in Table 1.

**Table 1**

| Ingredients | Concentration (%w/v) |
|---|---|
| Sodium hyaluronate | 0.1 |
| HP-Guar | 0.1 |
| Carbopol 974P | 0.1 |
| Mannitol | 4.0 |
| NaOH/HCl | qs to pH 7.0 |
| Purified water | qs to 100 |

The composition shown in Table 1 can be prepared by the following method. Add the following ingredients slowly and in the following order to heated purified water (70 - 80 °C) (approximately 80% of the desired batch volume) with mixing: mannitol, Carbopol 974P, HP-Guar and Sodium hyaluronate, (waiting until each ingredient is mixed well before adding the next). pH is then adjusted with 1 N NaOH, and the remaining amount of purified water is added and adjust to the final volume.

### Example 2: Synergistic Effect on Viscosity

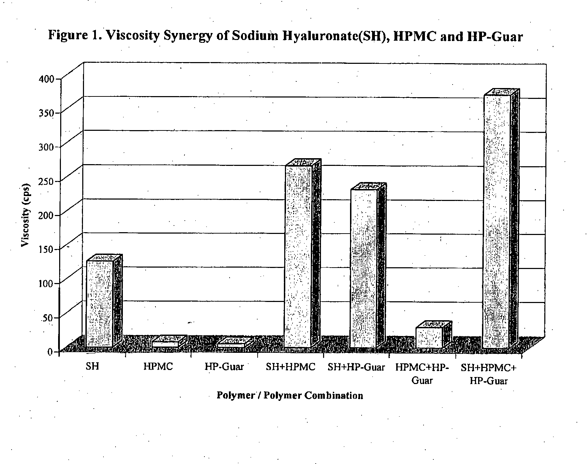

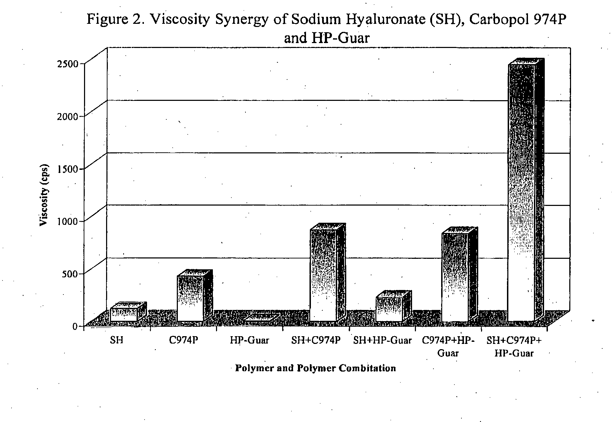

The compositions shown in Tables 2A and 2B were prepared and their viscosity determined using a Brookfield cone/plate viscometer with number 42 cone/plate set (30 rpm, at 25 °C) for less viscous samples (viscosity less than 20 cps) and (6 rpm, at 25 °C) for sample viscosities between 20 - 50 cps. Number 52 cone/plate set (6 rpm, at 25 °C) was used for more viscous samples (viscosity more than 50 cps). The results are shown in Tables 2A and 2B. The results are also shown in Figures 1 and 2.

**Table 2A**

| | Composition (%w/v) | | | | |
|---|---|---|---|---|---|
| Ingredient | 1 | 2 | 3 | 4 | 5 |
| Mannitol | 4.0 | 4.0 | 4.0 | 4.0 | 4.0 |
| Sodium Hyaluronate | --- | 0.1 | --- | --- | --- |
| HP-Guar | --- | --- | 0.1 | --- | --- |
| HPMC 2910 | --- | --- | --- | 0.3 | --- |
| Carbopol 974P | --- | --- | --- | --- | 0.1 |
| NaOH/HCl | q.s. pH 7.0 | q.s. pH 7.0 | q.s. pH 7.0 | q.s. pH 7.0 | q.s. pH 7.0 |
| Purified Water | q.s. 100 | q.s. 100 | q.s. 100 | q.s. 100 | q.s. 100 |
| Final pH | 7.06 | 6.93 | 6.99 | 7.06 | 6.96 |
| Viscosity (cps) | 1.1 | 125.9 | 5.6 | 8.0 | 432.3 |

**Table 2B**

| Ingredient | 6 | 7 | 8 | 9 | 10 | 12 | 13 |
|---|---|---|---|---|---|---|---|
| Mannitol | 4.0 | 4.0 | 4.0 | 4.0 | 4.0 | 4.0 | 4.0 |
| Sodium Hyaluronate | 0.1 | 0.1 | 0.1 | --- | --- | 0.1 | 0.1 |
| HP-Guar | 0.1 | --- | --- | 0.1 | 0.1 | 0.1 | 0.1 |
| HPMC 2910 | --- | 0.3 | --- | 0.3 | --- | 0.3 | --- |
| Carbopol 974P | --- | --- | 0.1 | --- | 0.1 | --- | 0.1 |
| NaOH/HCl | q.s. pH 7.0 | q.s. pH 7.0 | q.s. pH 7.0 | q.s. pH 7.0 | q.s. pH 7.0 | q.s. pH 7.0 | q.s. pH 7.0 |
| Purified Water | q.s. 100 | q.s. 100 | q.s. 100 | q.s. 100 | q.s. 100 | q.s. 100 | q.s. 100 |
| Final pH | 6.94 | 7.08 | 7.06 | 7.02 | 7.01 | 7.03 | 7.03 |
| Viscosity (cps) | 232.3 | 267 | 873.5 | 30.2 | 840.2 | 371.6^{@} | 2445^{@} |

| | | | | | | | |
|---|---|---|---|---|---|---|---|
| ^{@} substantial synergy: greater than 150% of the simple sum of the three respective single polymer solutions | | | | | | | |

### Example 3

The experiment described in Example 2 was repeated for the sodium hyaluronate, guar gum, and carbomer system. The results are shown in Table 3. Although the measured viscosities shown in Table 3 do not match exactly those shown in Tables 2A and 2B (likely due to lot-to-lot variations in raw materials, equipment variability, and differences in ages of samples), this experiment nevertheless confirms the synergistic effect on viscosity of the combination of sodium hyaluronate, guar gum, and carbomer.

**Table 3**

| **Sample** | **14** | **15** | **16** | **17** | **18** | **19** | **20** |
|---|---|---|---|---|---|---|---|
| **Descriptions** | | | | **Two Component System** | | | |
| Mannitol (%) | 4 | 4 | 4 | 4 | 4 | 4 | 4 |
| Sodium Hyaluronate | 0.1 | | | 0.1 | 0.1 | | 0.1 |
| Carbopol 974P (%) | | 0.1 | | 0.1 | | 0.1 | 0.1 |
| HP Guar | | | 0.1 | | 0.1 | 0.1 | 0.1 |
| pH | 7.0 | 7.0 | 7.0 | 7.0 | 7.0 | 7.0 | 7.0 |
| | | | | | | | |
| **To Make:** | | | | | | | |
| Mannitol (g) | 4 | 4 | 4 | 4 | 4 | 4 | 4 |
| Sodium Hyaluronate (g) | 0.1 | | | 0.1 | 0.1 | 0 | 0.1 |
| Carbopol 974P (g) | | 0.1 | | 0.1 | 0 | 0.1 | 0.1 |
| HP Guar (g) | | | 0.1 | 0 | 0.1 | 0.1 | 0.1 |
| **Total Volume (mL)** | **100** | **100** | **100** | **100** | **100** | **100** | **100** |
| | | | | | | | |
| **Physical Parameter** | | | | | | | |
| Final pH | 7.02 | 7.08 | 6.93 | 6.93 | 7.04 | 6.96 | 7.01 |
| **Mean Viscosity (cps) (n=2)** | **125.5** | **466.5** | **4.55** | **930** | **257.5** | **1184** | **3002** |
| | | | | | | | |
| **Viscosity Synergy Analysis** | | | | | | | |
| **% Viscosity Increased *** | **NA** | **NA** | **NA** | **157%** | **198%** | **251%** | **503%** |

| | | | | | | | |
|---|---|---|---|---|---|---|---|
| * - the change (%) in viscosity compared to the simple sum of the respective two or three polymer solutions. | | | | | | | |

### Example 4: Lack of Synergistic Effect on Viscosity (Polyvinyl Alcohol + Chondroitin Sulfate + Polyvinylpyrrolidone)

The compositions shown in Table 4 were prepared and their viscosity determined using a Brookfield cone/plate viscometer with number 42 cone/plate set (30 rpm, at 25 °C). Two people independently prepared the indicated samples and measured their viscosity values (n = 1) for each person. The averages of each set of results are shown in Table 4. Airvol 523S is a commercially available polyvinyl alcohol polymer. Chondroitin sulfate is a commercially available polymer. K90 is a commercially available polyvinylpyrrolidone polymer.

**Table 4**

| | Composition (% w/v) | | | | | | | |
|---|---|---|---|---|---|---|---|---|
| Ingredient | 21 | 22 | 23 | 24 | 25 | 26 | 27 | 28 |
| Mannitol | 4.0 | 4.0 | 4.0 | 4.0 | 4.0 | 4.0 | 4.0 | 4.0 |
| PVA (Airvol 523S) | --- | 0.2 | --- | --- | 0.2 | 0.2 | --- | 0.2 |
| Chondroitin Sulfate | --- | --- | 0.2 | --- | 0.2 | --- | 0.2 | 0.2 |
| PVP (K90) | --- | --- | --- | 0.2 | --- | 0.2 | 0.2 | 0.2 |
| NaOH/HCl | q.s. pH 7.0 | q.s. pH 7.0 | q.s. pH 7.0 | q.s. pH 7.0 | q.s. pH 7.0 | q.s. pH 7.0 | q.s. pH 7.0 | q.s. pH 7.0 |
| Purified Water | q.s. 100 | q.s. 100 | q.s. 100 | q.s. 100 | q.s. 100 | q.s. 100 | q.s. 100 | q.s. 100 |
| Viscosity (cps) | 1.0 | 1.5 | 1.3 | 1.4 | 1.7 | 1.9 | 1.8 | 2.3* |
| Subst. Synergy^{@} | --- | --- | --- | --- | --- | --- | --- | No |

| | | | | | | | | |
|---|---|---|---|---|---|---|---|---|
| * slight, transparent precipitate observed ^{@} Subst. Synergy = substantial synergy: greater than 150% of the simple sum of the three respective single polymer solutions | | | | | | | | |

### Example 5: Lack of Synergistic Effect on Viscosity (Polyvinyl Alcohol + Chondroitin Sulfate + Carbomer; Polyvinyl Alcohol + Polyvinylpyrrolidone + Carbomer; Chondroitin Sulfate + Polyvinylpyrrolidone + Carbomer)

The compositions shown in Table 5 were prepared and their viscosity determined using a Brookfield cone/plate viscometer with number 42 cone/plate set (30 rpm, at 25 °C) for less viscous samples (viscosity less than 20 cps) and number 52 cone/plate set (3 rpm, at 25 °C) for more viscous samples (viscosity more than 20 cps). Two people independently prepared the indicated samples and measured their viscosity values (n = 1) for each person. The averages of each set of results are shown in Table 5. Airvol 523S is a commercially available polyvinyl alcohol polymer. Chondroitin sulfate is a commercially available polymer. K90 is a commercially available polyvinylpyrrolidone polymer. The viscosities of the single polymer solutions for polyvinyl alcohol, chondroitin sulfate and polyvinylpyrrolidone can be found in Table 4 - Examples 20 - 22.

**Table 5**

| | Composition (% w/v) | | | | | | |
|---|---|---|---|---|---|---|---|
| Ingredient | 29 | 30 | 31 | 32 | 33 | 34 | 35 |
| Mannitol | 4.0 | 4.0 | 4.0 | 4.0 | 4.0 | 4.0 | 4.0 |
| PVA (Airvol 523S) | --- | 0.2 | --- | --- | --- | 0.2 | 0.2 |
| Chondroitin Sulfate | --- | --- | 0.2 | --- | 0.2 | 0.2 | --- |
| PVP (K90) | --- | --- | --- | 0.2 | 0.2 | --- | 0.2 |
| Carbopol 974P | 0.1 | 0.1 | 0.1 | 0.1 | 0.1 | 0.1 | 0.1 |
| NaOH/HCl | q.s. pH 7.0 | q.s. pH 7.0 | q.s. pH 7.0 | q.s. pH 7.0 | q.s. pH 7.0 | q.s. pH 7.0 | q.s. pH 7.0 |
| Purified Water | q.s. 100 | q.s. 100 | q.s. 100 | q.s. 100 | q.s. 100 | q.s. 100 | q.s. 100 |
| Viscosity (cps) | 441.6 | 323.8 | 12.7 | N/A* | 16.7** | 14.2 | N/A* |
| Subst. Synergy^{@} | --- | --- | --- | --- | --- | No | No |

| | | | | | | | |
|---|---|---|---|---|---|---|---|
| * PVP was incompatible with Carbopol 974P - it formed a precipitate. ** Solution obtained only by specific order of mixing: mannitol, then chondroitin sulfate, then PVP, then carbomer. ^{@} Subst. Synergy = substantial synergy: greater than 150% of the simple sum of the three respective single polymer solutions | | | | | | | |

### Example 6: Effect of Total Polymer Concentration on Viscosity

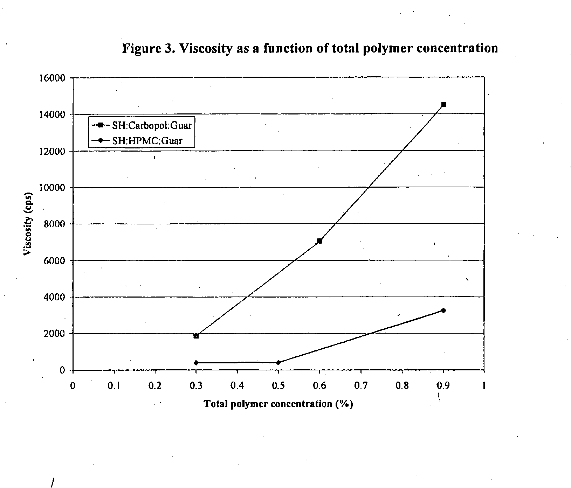

The effect of total polymer concentration on the viscosity of compositions containing a combination of HA, HP-Guar, and either Carbopol 974P or HPMC 2910 was evaluated using 6 compositions containing only the three designated polymers, mannitol and purified water. In each case, the composition contained 4.0 % (w/w) of mannitol and had an adjusted pH of 7.0. The total polymer concentrations ranged from 0.3 to 0.9. The viscosity was determined at 25 °C using a Brookfield viscometer (Model No. RVTDV-IICP #14797) @ 5 rpm. The results are shown in Tables 6A (sodium hyaluronate: hydroxypropyl methylcellulose:HP Guar) and 6B (sodium hyaluronate:carbopol:HP Guar). The results are also shown in Figure 3.

**Table 6A**

| | SH:HPMC:HP Guar | | |
|---|---|---|---|
| **Sample** | **1** | **2** | **3** |
| Mannitol (g) | 4 | 4 | 4 |
| Sodium Hyaluronate (g) | 0.1 | 0.1 | 0.3 |
| HPMC (g) | 0.1 | 0.3 | 0.3 |
| HP Guar (g) | 0.1 | 0.1 | 0.3 |
| pH | 7.0 | 7.0 | 7.0 |
| **Total Volume (mL)** | 100 | 100 | 100 |

| **Physical Parameter** | | | |
|---|---|---|---|
| **Final pH** | 7.0 | 7.0 | 7.0 |
| **Osmolality (mOsm/kg)** | 247 | 237 | 254 |
| **Viscosity (cps)** | **374** | **394** | **3240** |

**Table 6B**

| | SH:Carbopol:HP Guar | | |
|---|---|---|---|
| **Sample** | **4** | **5** | **6** |
| Mannitol (g) | 4 | 4 | 4 |
| Sodium Hyaluronate (g) | 0.1 | 0.2 | 0.3 |
| Carbopol 974P (g) | 0.1 | 0.2 | 0.3 |
| HP Guar (g) | 0.1 | 0.2 | 0.3 |
| pH | 7.0 | 7.0 | 7.0 |
| **Total Volume (mL)** | **100** | **100** | **100** |

| **Physical Parameter** | | | |
|---|---|---|---|
| **Final pH** | 7.0 | 7.0 | 7.0 |
| **Osmolality (mOsm/kg)** | 244 | 273 | * |
| **Viscosity (cps)** | **1850** | **7040** | **14500** |

| | | | |
|---|---|---|---|
| *Could not be determined due to high viscosity | | | |

The invention has been described by reference to certain preferred embodiments. .The embodiments described above are therefore considered to be illustrative in all respects.

## Claims

1. An aqueous composition suitable for ophthalmic administration comprising three polymeric ingredients having a synergistic effect on the composition's viscosity which is greater than 150% of the simple sum of the viscosities of the three respective simple polymer solutions and which is determined using a Brookfield cone/plate viscosimeter with number 42 cone/plate set (30 rpm, at 25°C) for samples with viscosity less than 20 cps and (6 rpm, at 25°C) for samples with viscosities between 20-50 cps and number 52 cone/plate set (6 rpm, at 25°C) for samples with viscosity more than 50 cps, wherein the three polymeric ingredients are a) hyaluronic acid, b) guar gum, and c) hydroxypropyl methylcellulose or a carboxyvinyl polymer, provided that if the composition comprises a carboxyvinyl polymer then the composition does not contain sodium chloride or boric acid, wherein the three polymeric ingredients are present in a ratio of 1:1:1 to 3:3:3, and the total concentration of the three polymeric ingredients ranges from 0.1 - 1% (w/v).

2. The composition of Claim 1 wherein the hyaluronic acid ingredient is sodium hyaluronate and the guar gum ingredient is hydroxypropyl guar.

3. The composition of Claim 1 wherein the composition comprises a carboxyvinyl polymer and the carboxyvinyl polymer is a polymer of acrylic acid crosslinked with allyl sucrose or allylpentaerythritol.

4. The composition of Claim 1 wherein the total concentration of the three polymeric ingredients ranges from 0.3 - 0.9% (w/v), preferably from 0.3 - 0.7% (w/v).

5. The composition of Claim 1 further comprising an ingredient selected from the group consisting of pharmaceutically acceptable buffering agents; preservatives; surfactants; solubilizing agents; emollients; pH-adjusting agents; and lubricants.

6. The composition of Claim 1 further comprising an ophthalmic drug, preferably an ophthalmic drug being selected from the group consisting of anti-glaucoma agents; anti-angiogenesis agents; anti-infective agents; non-steroidal and steroidal anti-inflammatory agents; growth factors; immunosuppressant agents; and anti-allergic agents.

7. The composition of Claim 1 for use as an artificial tear wherein the composition comprises a) hyaluronic acid; b) hydroxypropyl guar; c) hydroxypropyl methylcellulose or a carboxyvinyl polymer; and d) mannitol, wherein the composition has a pH of 6 - 8 and an osmolality in the range of 220 - 340 mOsm/kg.

8. Use of three polymeric ingredients for the manufacture of a topical ophthalmic composition for alleviating the symptoms of dry eye wherein three polymeric ingredients have a synergistic effect on the composition's viscosity which is greater than 150% of the simple sum of the viscosities of the three respective simple polymer solutions and which is determined using a Brookfield cone/plate viscosimeter with number 42 cone/plate set (30 rpm, at 25°C) for samples with viscosity less than 20 cps and (6 rpm, at 25°C) for samples with viscosities between 20-50 cps and number 52 cone/plate set (6 rpm, at 25°C) for samples with viscosity more than 50 cps, and wherein the three polymeric ingredients are a) hyaluronic acid, b) guar gum, and c) hydroxypropyl methyl cellulose or a carboxyvinyl polymer, provided that if the composition comprises a carboxyvinyl polymer then the composition does not contain sodium chloride or boric acid, wherein the three polymeric ingredients are present in a ratio of 1:1:1 to 3:3:3, and the total concentration of the three polymeric ingredients ranges from 0.1 - 1% (w/v).

9. The use of Claim 8 wherein the hyaluronic acid ingredient is sodium hyaluronate and the guar gum ingredient is hydroxypropyl guar.

10. The use of Claim 8 wherein the composition comprises a carboxyvinyl polymer and the carboxyvinyl polymer is a polymer of acrylic acid crosslinked with allyl sucrose or allylpentaerythritol.

11. The use of Claim 8 wherein the total concentration of the three polymeric ingredients ranges from 0.3 - 0.9% (w/v).

12. The use of Claim 8 wherein the composition comprises hyaluronic acid; hydroxypropyl guar; either hydroxypropyl methylcellulose or a carboxyvinyl polymer; and mannitol, wherein the composition has a pH of 6 - 8 and an osmolality in the range of 235 - 300 mOsm/kg.

13. Use of an ophthalmic drug and a carrier for the manufacture of an ophthalmic composition for treating a disease or condition of the eye wherein the carrier comprises three polymeric ingredients having a synergistic effect on the composition's viscosity which is greater than 150% of the simple sum of the viscosities of the three respective simple polymer solutions and which is determined using a Brookfield cone/plate viscosimeter with number 42 cone/plate set (30 rpm, at 25°C) for samples with viscosity less than 20 cps and (6 rpm, at 25°C) for samples with viscosities between 20-50 cps and number 52 cone/plate set (6 rpm, at 25°C) for samples with viscosity more than 50 cps, and wherein the three polymeric ingredients are a) hyaluronic acid, b) guar gum, and c) hydroxypropyl methylcellulose or a carboxyvinyl polymer, provided that if the composition comprises a carboxyvinyl polymer then the composition does not contain sodium chloride or boric acid, wherein the three polymeric ingredients are present in a ratio of 1:1:1 to 3:3:3, and the total concentration of the three polymeric ingredients ranges from 0.1 - 1% (w/v).

14. The use of Claim 13 wherein the composition is adapted for topical administration to the eye.

15. The use of Claim 13 wherein the composition is an injection, preferably an intravitreal injection, subconjunctival injection, sub-tenon injection, retrobulbar injection, suprachoroidal injection, or periocular injection.

## Patentansprüche

1. Wässerige Zusammensetzung, die für die ophthalmische Verabreichung geeignet ist, umfassend drei polymere Bestandteile mit einem synergistischen Effekt auf die Viskosität der Zusammensetzung, welche größer als 150 % der einfachen Summe der Viskositäten der drei jeweiligen einfachen Polymerlösungen ist und welche unter Verwendung eines Brookfield-Kegel/Platte-Viskosimeters mit dem Kegel/Platte-Satz Nummer 42 (30 U/min., bei 25 °C) für Proben mit einer Viskosität von weniger als 20 cP und (6 U/min., bei 25 °C) für Proben mit Viskositäten zwischen 20 und 50 cP und dem Kegel/Platte-Satz Nummer 52 (6 U/min., bei 25 °C) für Proben mit einer Viskosität von mehr als 50 cP bestimmt wird, wobei die drei polymeren Bestandteile a) Hyaluronsäure, b) Guargummi und c) Hydroxypropylmethylcellulose oder ein Carboxyvinylpolymer sind, mit der Maßgabe, dass, wenn die Zusammensetzung ein Carboxyvinylpolymer umfasst, dann die Zusammensetzung weder Natriumchlorid noch Borsäure enthält, wobei die drei polymeren Bestandteile in einem Verhältnis von 1 : 1 : 1 bis 3 : 3 : 3 vorliegen und die Gesamtkonzentration der drei polymeren Bestandteile im Bereich von 0,1 - 1 % (Gew./Vol.) liegt.

2. Zusammensetzung nach Anspruch 1, wobei der Hyaluronsäure-Bestandteil Natriumhyaluronat ist und der Guargummi-Bestandteil Hydroxypropylguar ist.

3. Zusammensetzung nach Anspruch 1, wobei die Zusammensetzung ein Carboxyvinylpoly-mer umfasst und das Carboxyvinylpolymer ein Polymer von Acrylsäure, vernetzt mit Allylsaccharose oder Allylpentaerythritol, ist.

4. Zusammensetzung nach Anspruch 1, wobei die Gesamtkonzentration der drei polymeren Bestandteile im Bereich von 0,3 - 0,9 % (Gew./Vol.) liegt, vorzugsweise 0,3 - 0,7 % (Gew./Vol.) beträgt.

5. Zusammensetzung nach Anspruch 1, ferner umfassend einen Bestandteil, ausgewählt aus der Gruppe, bestehend aus pharmazeutisch akzeptablen Puffersubstanzen; Konservierungsmitteln; oberflächenaktiven Mitteln; Lösungsvermittlern; Erweichungsmitteln; pH-Einstellmitteln und Schmiermitteln.

6. Zusammensetzung nach Anspruch 1, ferner umfassend ein ophthalmisches Arzneimittel, vorzugsweise ein ophthalmisches Arzneimittel, das aus der Gruppe ausgewählt ist, bestehend aus Antiglaukommitteln; Antiangiogenesemitteln; Antiinfektiva; nicht steroidalen und steroidalen Entzündungshemmern; Wachstumsfaktoren; Immunosuppressiva und antiallergischen Mitteln.

7. Zusammensetzung nach Anspruch 1 zur Verwendung als künstliche Tränenflüssigkeit, wobei die Zusammensetzung a) Hyaluronsäure; b) Hydroxypropylguar; c) Hydroxypropylmethylcellulose oder ein Carboxyvinylpolymer und d) Mannitol umfasst, wobei die Zusammensetzung einen pH von 6 - 8 und eine Osmolalität im Bereich von 220 - 340 mOsm/kg hat.

8. Verwendung von drei polymeren Bestandteilen zur Herstellung einer topischen ophthalmischen Zusammensetzung zur Linderung der Symptome trockener Augen, wobei die drei polymeren Bestandteile einen synergistischen Effekt auf die Viskosität der Zusammensetzung haben, welche größer als 150 % der einfachen Summe der Viskositäten der drei jeweiligen einfachen Polymerlösungen ist und welche unter Verwendung eines Brookfield-Kegel/Platte-Viskosimeters mit dem Kegel/Platte-Satz Nummer 42 (30 U/min., bei 25 °C) für Proben mit einer Viskosität von weniger als 20 cP und (6 U/min., bei 25 °C) für Proben mit Viskositäten zwischen 20 und 50 cP und dem Kegel/Platte-Satz Nummer 52 (6 U/min., bei 25 °C) für Proben mit einer Viskosität von mehr als 50 cP bestimmt wird, und wobei die drei polymeren Bestandteile a) Hyaluronsäure, b) Guargummi und c) Hydroxypropylmethylcellulose oder ein Carboxyvinylpolymer sind, mit der Maßgabe, dass, wenn die Zusammensetzung ein Carboxyvinylpolymer umfasst, dann die Zusammensetzung weder Natriumchlorid noch Borsäure enthält, wobei die drei polymeren Bestandteile in einem Verhältnis von 1 : 1 : 1 bis 3 : 3 : 3 vorliegen und die Gesamtkonzentration der drei polymeren Bestandteile im Bereich von 0,1 - 1 % (Gew./Vol.) liegt.

9. Verwendung nach Anspruch 8, wobei der Hyaluronsäure-Bestandteil Natriumhyaluronat ist und der Guargummi-Bestandteil Hydroxypropylguar ist.

10. Verwendung nach Anspruch 8, wobei die Zusammensetzung ein Carboxyvinylpolymer umfasst und das Carboxyvinylpolymer ein Polymer von Acrylsäure, vernetzt mit Allylsaccharose oder Allylpentaerythritol, ist.

11. Verwendung nach Anspruch 8, wobei die Gesamtkonzentration der drei polymeren Bestandteile im Bereich von 0,3 - 0,9 % (Gew./Vol.) liegt.

12. Verwendung nach Anspruch 8, wobei die Zusammensetzung Hyaluronsäure; Hydroxypropylguar; entweder Hydroxypropylmethylcellulose oder ein Carboxyvinylpolymer und Mannitol umfasst, wobei die Zusammensetzung einen pH von 6 - 8 und eine Osmolalität im Bereich von 235 - 300 mOsm/kg hat.

13. Verwendung eines ophthalmischen Arzneimittels und eines Trägers zur Herstellung einer ophthalmischen Zusammensetzung zur Behandlung einer Erkrankung oder eines Zustandes der Augen, wobei der Träger drei polymere Bestandteile mit einem synergistischen Effekt auf die Viskosität der Zusammensetzung umfasst, welche größer als 150 % der einfachen Summe der Viskositäten der drei jeweiligen einfachen Polymerlösungen ist und welche unter Verwendung eines Brookfield-Kegel/Platte-Viskosimeters mit dem Kegel/Platte-Satz Nummer 42 (30 U/min., bei 25 °C) für Proben mit einer Viskosität von weniger als 20 cP und (6 U/min., bei 25 °C) für Proben mit Viskositäten zwischen 20 und 50 cP und dem Kegel/Platte-Satz Nummer 52 (6 U/min., bei 25 °C) für Proben mit einer Viskosität von mehr als 50 cP bestimmt wird, und wobei die drei polymeren Bestandteile a) Hyaluronsäure, b) Guargummi und c) Hydroxypropylmethylcellulose oder ein Carboxyvinylpolymer sind, mit der Maßgabe, dass, wenn die Zusammensetzung ein Carboxyvinylpolymer umfasst, dann die Zusammensetzung weder Natriumchlorid noch Borsäure enthält, wobei die drei polymeren Bestandteile in einem Verhältnis von 1 : 1 : 1 bis 3 : 3 : 3 vorliegen und die Gesamtkonzentration der drei polymeren Bestandteile im Bereich von 0,1 - 1 % (Gew./Vol.) liegt.

14. Verwendung nach Anspruch 13, wobei die Zusammensetzung für die topische Verabreichung an das Auge angepasst ist.

15. Verwendung nach Anspruch 13, wobei die Zusammensetzung eine Injektion, vorzugsweise eine Glaskörperinjektion, subkonjunktivale Injektion, Subtenon-Injektion, retrobulbäre Injektion, suprachoroidale Injektion oder periokuläre Injektion ist.

## Revendications

1. Une composition aqueuse destinée à une administration ophtalmique comprenant trois ingrédients polymères présentant un effet synergique sur la viscosité de la composition qui est supérieure à 150 % de la simple somme des viscosités des trois solutions polymères simples respectivement et qui est déterminée en utilisant un viscosimètre à cône/à plaque de type Brookfield avec un nombre de 42 ensembles de plaque/cône (30 tpm, à 25 °C) pour des échantillons avec une viscosité inférieure à 20 cps et (6 tpm, à 25 °C) pour des échantillons avec des viscosités entre 20 - 50 cps et avec un nombre de 52 ensembles de plaque/cône (6 rpm, à 25 °C) pour des échantillons avec une viscosité supérieure à 50 cps, dans laquelle les trois ingrédients polymères sont a) l'acide hyaluronique, b) la gomme de guar, et c) le méthylcellulose d'hydroxypropyle ou un polymère de carboxyvinyle, pour autant que si la composition comprend un polymère de carboxyvinyle alors la composition ne contient pas de chlorure de sodium ou d'acide borique, dans laquelle les trois ingrédients polymères sont présents dans un rapport de 1 :1 :1 à 3 :3 :3, et la concentration totale des trois ingrédients polymères est comprise dans la plage de 0,1 à 1 % (poids, volume).

2. La composition selon revendication 1, dans laquelle l'ingrédient acide hyaluronique est de l'hyaluronate de sodium et l'ingrédient gomme de guar est du guar d'hydroxypropyle.

3. La composition selon la revendication 1, dans laquelle la composition comprend un polymère de carboxyvinyle et le polymère de carboxyvinyle est un polymère d'acide acrylique réticulé avec du sucrose allylique ou du pentaérythritol allylique.

4. La composition selon la revendication 1, dans laquelle la concentration totale des trois ingrédients polymères est comprise dans la plage de 0,3 à 0,9 % (poids, volume), de préférence de 0,3 - 0,7 % (poids, volume).

5. La composition selon la revendication 1, comprenant de plus un ingrédient choisi dans le groupe constitué ; d'agents de conservation ; de tensioactifs, d'agents de solubilisation ; d'émollients ; d'agent d'ajustement de pH ; et de lubrifiants et d'agents tampon pharmaceutiquement acceptables.

6. La composition selon la revendication 1, comprenant de plus un médicament ophtalmique, de préférence un médicament ophtalmique étant choisi dans le groupe constitué d'agents anti-glaucomes; d'agents anti-angiogenèses ; d'agents anti-infectieux ; d'agents anti-inflammatoires stéroïdiens et non-stéroïdiens ; de facteurs de croissance ; d'agents immunosuppresseurs et d'agents antiallergiques.

7. La composition selon la revendication 1 pour une utilisation en tant que larme artificielle dans laquelle la composition comprend a) de l'acide hyaluronique; b) du guar d'hydroxypropyle; c) la méthylcellulose d'hydroxypropyle ou un polymère de carboxyvinyle; et d) du mannitol, dans laquelle la composition présente un pH de 6-8 et une osmolalité située dans la plage de 220 à 340 mOsm/kg.

8. Utilisation de trois ingrédients polymères pour la fabrication d'une composition ophtalmique topique pour atténuer les symptômes de sécheresse oculaire dans laquelle les trois ingrédients polymère présentant un effet synergique sur la viscosité de la composition qui est supérieure à 150 % de la simple somme des viscosités des trois solutions polymères simples respectivement et qui est déterminée en utilisant un viscosimètre à cône/à plaque de type Brookfield avec un nombre de 42 ensembles de plaque/cône (30 tpm, à 25 °C) pour des échantillons avec une viscosité inférieure à 20 cps et (6 tpm, à 25 °C) pour des échantillons avec des viscosités entre 20 - 50 cps et avec un nombre de 52 ensembles de plaque/cône (6 rpm, à 25 °C) pour des échantillons avec une viscosité supérieure à 50 cps, dans laquelle les trois ingrédients polymères sont a) l'acide hyaluronique, b) la gomme de guar, et c) le méthylcellulose d'hydroxypropyle ou un polymère de carboxyvinyle, à condition que si la composition comprend un polymère de carboxyvinyle alors la composition ne contient pas de chlorure de sodium ou d'acide borique, dans laquelle les trois ingrédients polymères sont présents dans un rapport de 1 :1 :1 à 3 :3 :3, et la concentration totale des trois ingrédients polymères sont compris dans la plage allant de 0,1 à 1 % (poids, volume).

9. Utilisation selon la revendication 8, dans laquelle l'ingrédient d'acide hyaluronique est de l'hyaluronate de sodium et l'ingrédient de gomme de guar est du guar d'hydroxypropyle.

10. Utilisation selon la revendication 8, dans laquelle la composition comprend un polymère de carboxyvinyle et le polymère de carboxyvinyle est un polymère d'acide acrylique réticulé avec du sucrose allylique ou du pentaérythritol allylique.

11. Utilisation selon la revendication 8, dans laquelle la concentration totale des trois ingrédients polymères se situe dans la plage allant de 0,3 à 0,9 % (poids, volume).

12. Utilisation selon la revendication 8, dans laquelle la composition comprend de l'acide hyaluronique; du guar d'hydroxypropyle soit de la méthylcellulose d'hydroxypropyle soit un polymère de carboxyvinyle ; et du mannitol, dans laquelle la composition présente un pH de 6-8 et une osmolalité située dans la plage de 235 à 300 mOsm/kg.

13. Utilisation d'un médicament ophtalmique et d'un support la fabrication d'une composition ophtalmique pour traiter une maladie ou une affection de l'oeil dans laquelle la support comprend trois ingrédients polymères présentant un effet synergique sur la viscosité de la composition qui est supérieure à 150 % de la simple somme des viscosités des trois solutions polymères simples respectivement et qui est déterminée en utilisant un viscosimètre à cône/à plaque de type Brookfield avec un nombre de 42 ensembles de plaque/cône (30 tpm, à 25 °C) pour des échantillons avec une viscosité inférieure à 20 cps et (6 tpm, à 25 °C) pour des échantillons avec des viscosités entre 20 - 50 cps et avec un nombre de 52 ensembles de plaque/cône (6 rpm, à 25 °C) pour des échantillons avec une viscosité supérieure à 50 cps, dans laquelle les trois ingrédients polymères sont a) l'acide hyaluronique, b) la gomme de guar, et c) le méthylcellulose d'hydroxypropyle ou un polymère de carboxyvinyle, à condition que si la composition comprend un polymère de carboxyvinyle alors la composition ne contient pas de chlorure de sodium ou d'acide borique, dans laquelle les trois ingrédients polymères sont présents dans un rapport de 1 :1 :1 à 3 :3 :3, et la concentration totale des trois ingrédients polymères sont compris dans la plage allant de 0,1 à 1 % (poids, volume).

14. Utilisation selon la revendication 13, dans laquelle la composition est destinée à une administration topique dans l'oeil.

15. Utilisation selon la revendication 13, dans laquelle la composition est une composition d'injection, de préférence d'injection intravitreuse, d'injection subconjonctivale, d'injection sous-ténonienne, d'injection rétrobulbaire, d'injection suprachoroïdien ou d'injection périoculaire.
